# EUROPEAN PATENT SPECIFICATION

(11) **EP 4 034 187 B1**
(45) Date of publication and mention of the grant of the patent: **01.01.2025**
(21) Application number: 20789757.0
(22) Date of filing: 24.09.2020
(51) Int. Cl.: A61M 1/16, A61M 1/36, A61M 25/00, A61M 60/113, A61M 60/216, A61M 60/279, A61M 60/38, A61M 60/857, A61M 60/859

(54) **DUAL LUMEN DRAINAGE CANNULA WITH SINGLE OUTLET**
DOPPELLUMIGE DRAINAGEKANÜLE MIT EINFACHEM AUSLASS
CANULE DE DRAINAGE À DOUBLE LUMIÈRE AYANT UNE SORTIE UNIQUE

(30) Priority: 26.09.2019 US 201962906548 P
(43) Date of publication of application: 03.08.2022
(73) Proprietor: CardiacAssist, Inc., Pittsburgh, PA 15238 (US)
(72) Inventor: EDWARDS, Kelli, Glenshaw, Pennsylvania 15116 (US); KELLY, Patrick A., North Huntingdon, Pennsylvania 15642 (US); LINEHAN, Michael J., Pittsburgh, Pennsylvania 15201 (US)
(74) Representative: Buzzi, Notaro & Antonielli d'Oulx S.p.A.
(86) International application number: PCT/US2020/052468
(87) International publication number: WO 2021/061964

(56) References cited:
- WO-A1-2016/054543
- WO-A1-2018/013644
- US-B1- 6 508 777

## Description

### CROSS REFERENCE TO RELATED APPLICATIONS

The present application claims the benefit of and priority to US Provisional Patent Application Serial No. 62/906,548, filed on September 26, 2019, titled Dual Lumen Drainage Cannula With Single Outlet.

### BACKGROUND

### Field

The present disclosure generally relates to devices and methods for assisting a patient's heart with a cannula. More specifically, the present disclosure is related to cannula assemblies, systems including at least one cannula assembly, and methods of use thereof for medical procedures such as veno-arterial extracorporeal membrane oxygenation.

### Description of the Related Art

Veno-arterial extracorporeal membrane oxygenation (VA ECMO) is one method for treating right ventricular failure and respiratory failure percutaneously. A VA ECMO procedure draws blood from the right atrium and pumps it through an oxygenator and back into the arterial circulation via the femoral artery. VA ECMO bypasses the lungs and the heart completely, elevating arterial pressure and infusing blood into the arterial system with added oxygen and reduced carbon dioxide. One of the results of this therapy is that the blood that remains in the heart must be pumped by the heart to a higher pressure level in order to be ejected by the left ventricle because the VA ECMO system has elevated the arterial pressure to a higher level that represents a higher afterload to the pumping effort of the left ventricle.

In conventional VA ECMO systems, one drainage cannula is placed in the superior vena cava (SVC), the inferior vena cava (IVC), right atrium region by way of a femoral vein (typically) to drain blood therefrom and a separate, second return cannula is placed in an artery to return oxygenated (and cleansed from carbon dioxide) blood at a higher pressure. To drain additional blood from the pulmonary artery in conventional VA ECMO systems requires the insertion of a second drainage cannula (third total cannula) placed into the pulmonary artery by way of the jugular vein or other access site. Among the benefits of drawing blood from both the right atrium and the pulmonary artery is that the blood drained is fully mixed venous blood, including coronary circulation which drains into the right atrium, and that the right ventricle is unloaded to a greater extent. The use of multiple cannulas, however, consequently requires multiple cannula insertion sites, thereby increasing the risk of bleeding, vessel damage, and infection, as well as pain and discomfort to the patient.

While multi-lumen cannulas exist in the art, such cannulas may not be configured for draining blood flow from two separate sites. For example, a dual lumen cannula described in U.S. Patent Nos. 9,168,352, 9,782,534, and 10,279,101 can be used to unload the right side of the heart by drawing blood from the right atrium through one lumen and infusing blood to the pulmonary artery through a second lumen.

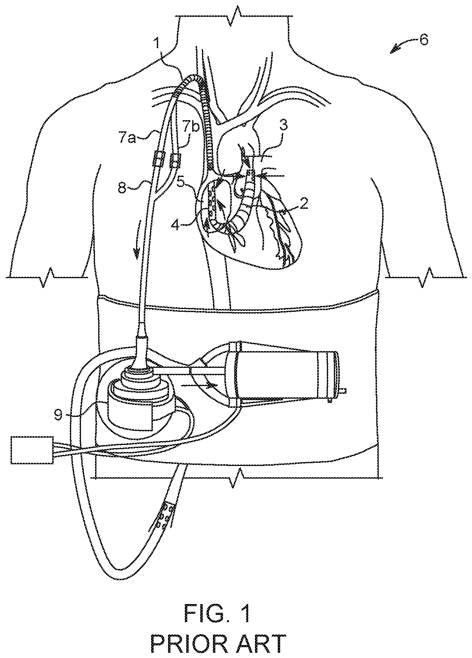

A more recent innovation in VA ECMO systems utilizes a dual lumen cannula in which both lumens are used for drainage to the pump. Such a system is described in PCT Patent Application Publication No. WO 2016/054543. However, in the VA ECMO systems described in that document, the lumens of the cannula are separated by a "Y" connector into two outlets, which must be rejoined by a separate connector element to create a single lumen of flow into the pump. An example of one such prior art VA ECMO system is shown in **FIG. 1****,** which illustrates a drainage cannula **1** inserted into the vasculature of a patient **6.** The cannula **1** includes a first tube **2** of which an opening thereof is positioned in the pulmonary artery **3,** and a second tube **4** of which an opening thereof is positioned in the right atrium **5** of the heart. The first tube **2** and the second tube **4** are fluidly separated from one another throughout the entirety of the cannula **1,** and each of the first tube **2** and the second tube **4** drain blood toward separate outlets **7a, 7b** of the cannula **1.** Both outlets **7a, 7b** of the cannula **1** are connected to a separate component, namely a Y-connector **8,** which joins the flow from the two outlets **7a, 7b** of the cannula **1** into a single flow path into a blood pump **9.** In such VA ECMO systems, separation of the cannula **1** into two outlets **7a, 7b** necessitates the use of the Y-connector **8.** The separate outlets **7a, 7b** allow for individual flow control through the first and second tubes **2, 4,** for example by providing a valve at each outlet **7a, 7b.** However, the necessitation of the Y-connector **8** to rejoin the flow of the separate outlets **7a, 7b** may be disadvantageous in some circumstances.

### SUMMARY

In view of the foregoing, there exists a need for a dual lumen cannula, particularly for use in VA ECMO procedures, capable of draining blood from multiple vascular locations while having a single outlet lumen for supplying the drained blood to a pump. Embodiments of the present disclosure are generally directed to a VA ECMO system, a cannula assembly for a VA ECMO system, and a method of providing VA ECMO of a heart.

Embodiments of the present disclosure are directed to a veno-arterial extracorporeal membrane oxygenation (VA ECMO) system including a dual lumen drainage cannula. The dual lumen drainage cannula includes a first drainage tube having a proximal end, a distal end, and at least one aperture defined in the distal end; a second drainage tube having a proximal end, a distal end, and at least one aperture defined in the distal end; and an outlet fitting comprising a single lumen in fluid communication with the first drainage tube and the second drainage tube. The system further includes a blood pump having an inlet connected to the outlet fitting of the dual lumen drainage cannula, an oxygenator connected to an outlet of the blood pump, and an infusion cannula connected to an outlet of the oxygenator and configured for insertion into the vasculature of a patient. The distal end of the second drainage tube is joined to a portion of the first drainage tube between the proximal and distal ends of the first drainage tube.

In some embodiments, the infusion cannula is configured for insertion into a femoral artery of the patient.

In some embodiments, the at least one aperture of the first drainage tube is configured for draining blood from a pulmonary artery of the patient, and the at least one aperture of the second drainage tube is configured for draining blood from a right atrium of the patient.

In some embodiments, the first drainage tube extends coaxially relative to the second drainage tube.

In some embodiments, the proximal end of the first drainage tube is positioned distally of the outlet fitting.

In other embodiments, the present disclosure is directed to a dual lumen drainage cannula assembly configured for use in a veno-arterial extracorporeal membrane oxygenation (VA ECMO) system. The dual lumen drainage cannula includes a first drainage tube having a proximal end, a distal end, and at least one aperture defined in the distal end. The dual lumen drainage cannula further includes a second drainage tube having a proximal end, a distal end, and at least one aperture defined in the distal end. The dual lumen drainage cannula further includes an outlet fitting comprising a single lumen in fluid communication with the first drainage tube and the second drainage tube. The distal end of the second drainage tube is joined to a portion of the first drainage tube between the proximal and distal ends of the first drainage tube.

In some embodiments, the dual lumen drainage cannula further includes a retainer for indexing the proximal end of the first drainage tube within the proximal end of the second drainage tube.

In some embodiments, the at least one aperture of the first drainage tube is configured for draining blood from a pulmonary artery of a patient, and the at least one aperture of the second drainage tube is configured for draining blood from a right atrium of the patient.

In some embodiments, the first drainage tube extends coaxially relative to the second drainage tube.

In some embodiments, the proximal end of the first drainage tube is positioned distally of the outlet fitting.

Other embodiments of the present disclosure are directed to a method of providing veno-arterial extracorporeal membrane oxygenation (VA ECMO) of a heart. The method includes providing a dual lumen drainage cannula including a first drainage tube having a proximal end, a distal end, and at least one aperture defined in the distal end; a second drainage tube having a proximal end, a distal end, and at least one aperture defined in the distal end; and an outlet fitting comprising a single lumen in fluid communication with the first drainage tube and the second drainage tube. The distal end of the second drainage tube is joined to a portion of the first drainage tube between the proximal and distal ends of the first drainage tube. The method further includes inserting the dual lumen drainage cannula into a first site in a patient's vasculature, maneuvering the dual lumen drainage cannula through the patient's vasculature such that the distal end of the first drainage tube is at least within proximity of the patient's pulmonary artery and such that the distal end of the second drainage tube is at least within proximity of the patient's right atrium, draining blood through the first drainage tube and the second drainage tube to a blood pump, pumping drained blood through an oxygenator to reduce carbon dioxide content of the blood, and delivering oxygenated blood with reduced carbon dioxide content to a second site in the patient's vasculature.

In some embodiments, the outlet fitting of the dual lumen drainage cannula is connected to an inlet of the blood pump.

In some embodiments, the first drainage tube extends coaxially relative to the second drainage tube.

In some embodiments, the dual lumen drainage cannula further includes a retainer for indexing the proximal end of the first drainage tube within the proximal end of the second drainage tube.

In some embodiments, the proximal end of the first drainage tube is positioned distally of the outlet fitting.

Further details and advantages of the present disclosure will be understood from the following detailed description read in conjunction with the accompanying drawings. The invention is defined by the appended claims.

### BRIEF DESCRIPTION OF THE DRAWINGS

**FIG. 1** is a schematic view of VA ECMO system as known in the prior art;
**FIG. 2** is a side view of a drainage cannula according to an embodiment of the present disclosure;
**FIG. 3** is a side view of the drainage cannula of **FIG. 2****,** with the second drainage tube removed for clarity;
**FIG. 4A** is a cross-sectional view of the drainage cannula taken alone line **II-II** of **FIG. 2****,** according to an embodiment of the present disclosure;
**FIG. 4B** is a cross-sectional view of the drainage cannula taken alone line **II-II** of **FIG. 2****,** according to another embodiment of the present disclosure;
**FIG. 4C** is a cross-sectional view of the drainage cannula taken alone line **II-II** of **FIG. 2****,** according to a further embodiment of the present disclosure;
**FIG. 5** is a cross-sectional view of detail **A** shown in **FIG. 3****,** according to an embodiment of the present disclosure;
**FIG. 6** is a cross-sectional view of the second drainage tube of the drainage cannula of **FIG. 3****;**
**FIG. 7** is a cross-sectional view of detail **B** in **FIG. 6****;**
**FIG. 8** is a cross-sectional view of the drainage cannula taken along line **I-I** of **FIG. 2****;**
**FIG. 9** is a cross-sectional view of detail **C** in **FIG. 8****,** illustrating a transition portion at a distal end of a drainage cannula of the drainage cannula;
**FIG. 10** is a schematic view of the drainage cannula of any of **FIGS. 2-9** positioned in the superior vena cava of a body of a patient;
**FIG. 11** is a schematic view of the drainage cannula of any of **FIGS. 2-9** positioned inside a patient's heart;
**FIG. 12** is a schematic view of a VA ECMO system, including the drainage cannula of any of **FIGS. 2-9****,** according to an embodiment of the present disclosure; and
**FIG. 13** is a cross-sectional view of the drainage cannula taken alone line **III-III** of **FIG. 2****,** according to an embodiment of the present disclosure.

### DETAILED DESCRIPTION

For purposes of the description hereinafter, the terms "end," "upper," "lower," "right," "left," "vertical," "horizontal," "top," "bottom," "lateral," "longitudinal," and derivatives thereof shall relate to the invention as it is oriented in the drawing figures. However, it is to be understood that the invention may assume various alternative variations and step sequences, except where expressly specified to the contrary. It is also to be understood that the specific devices and processes illustrated in the attached drawings, and described in the following specification, are simply exemplary embodiments or aspects. Hence, specific dimensions and other physical characteristics related to the embodiments or aspects disclosed herein are not to be considered as limiting.

As used herein, the term "at least one of" is synonymous with "one or more of'. For example, the phrase "at least one of A, B, and C" means any one of A, B, and C, or any combination of any two or more of A, B, and C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of'. For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F. When used in relation to a cannula, catheter, or other device inserted into a patient, the term "proximal" refers to a portion of such device farther from the end of the device inserted into the patient. When used in relation to a cannula, catheter, or other device inserted into a patient, the term "distal" refers to a portion of such device nearer to the end of the device inserted into the patient.

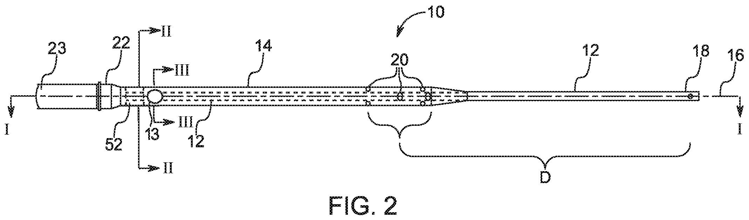

Referring to the drawings, in which like reference characters refer to like parts throughout the several views thereof, various embodiments of a dual lumen drainage cannula **10** (hereinafter referred to as "the drainage cannula **10**") are shown. With initial reference to **FIG. 2****,** the assembled drainage cannula **10,** according to one embodiment, generally includes a first drainage tube **12** having a first length and a second drainage tube **14** having a second length. The first length of the first drainage tube **12** is greater than the second length of the second drainage tube **14.** The first drainage tube **12** is disposed partially within the second drainage tube **14,** such that both the first drainage tube **12** and the second drainage tube **14** extend generally parallel to a central axis **16.** In some embodiments, the first drainage tube **12** and the second drainage tube **14** may be arranged coaxially with one another about the central axis **16.** An inner diameter of the second drainage tube **14** may be greater than an outer diameter of the first drainage tube **12** such that a flow cavity is formed inside the second drainage tube **14** around a portion of the first drainage tube **12** disposed within the second drainage tube **14.** The first drainage tube **12** and the second drainage tube **14** are fluidly separated from one another along the entire length of the first drainage tube **12,** such that a first fluid (e.g. blood drained from the pulmonary artery of a patient) carried through the first drainage tube **12** does not mix with a second fluid (e.g. blood drained from the right atrium of the patient) carried through the second drainage tube **14** until the first fluid reaches a proximal end of the first drainage tube **12.**

One or both of the first drainage tube **12** and the second drainage tube **14** may be manufactured from a medical-grade material such as polyurethane. Alternatively, the tubes may be made from PVC or silicone, and may be dip molded, extruded, co-molded, or made using any other suitable manufacturing technique.

With continued reference to **FIG. 2****,** a plurality of apertures **18** is provided at a distal end of the first drainage tube **12.** The plurality of apertures **18** is desirably arranged in a circular pattern extending around a circumference of the first drainage tube **12.** In some embodiments, the plurality of apertures **18** may be disposed in multiple groups provided at various sites on the first drainage tube **12.** Similarly, a plurality of apertures **20** are provided at a distal end of the second drainage tube **14.** The plurality of apertures **20** is desirably arranged in a circular pattern extending around the outer circumference of the second drainage tube **14.** In alternative embodiments, the plurality of drainage apertures **20** may be arranged in groups disposed at various sites along the length of the second drainage tube **14.** The apertures **18** of the first drainage tube **12** are separated along the length of the drainage cannula **10** from the apertures **20** of the second drainage tube **14** by a distance **D.** In some embodiments the distance **D** may be, or may correspond to, a vascular distance between the right atrium and the pulmonary artery of the patient such that the drainage cannula **10,** when positioned in a patient for a VA ECMO procedure, may drain blood from the pulmonary artery via the apertures **18** of the first drainage tube **12** and drain blood from the right atrium via the apertures 20 **of** the second drainage tube **14.** The distance **D** may vary based on the age and size of the patient, as well as the desired flow rates during the VA ECMO procedure. In some embodiments, the distance **D** may be, or may correspond to, a vascular distance between the right ventricle and the pulmonary artery of the patient such that the drainage cannula **10,** when positioned in a patient for a VA ECMO procedure, may drain blood from the pulmonary artery via the apertures **18** of the first drainage tube **12** and drain blood from the right ventricle via the apertures **20** of the second drainage tube **14.** In yet other embodiments, the apertures **20** may be positioned so as to drain blood from both the right atrium and the right ventricle simultaneously.

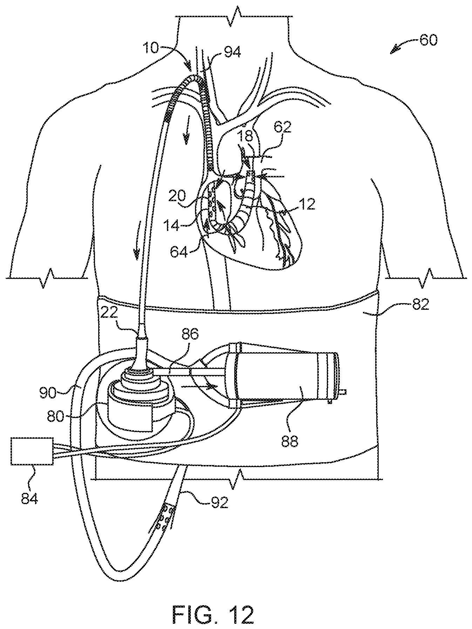

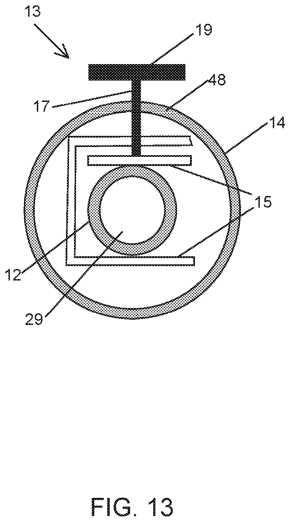

With continuing reference to **FIG. 2****,** an outlet fitting **22** may be provided at the proximal end of the drainage cannula **10** for connecting the drainage cannula **10** to other medical devices, such as a blood pump **80** (see **FIG. 12**). The outlet fitting **22** is in fluid communication with the first drainage tube **12** and the second drainage tube **14** such that the drainage cannula **10** defines only a single outlet for draining fluid from both the first drainage tube **12** and the second drainage tube **14.** In particular, the outlet fitting **22** defines a single outlet lumen **23** in fluid communication with the lumen of the first drainage tube **12** and the lumen of the second drainage tube **14** such that all flow in a proximal direction out of the drainage cannula **10** must flow through the single outlet lumen **23.** The outlet fitting **22** may be, for example, a male hose barb, a luer connector, a male or female threaded connector, or a continuation of the second drainage tube **14** configured to fit over a hose barb. With continuing reference to **FIG. 2****,** in some embodiments a valve **13** may be provided on the first drainage tube **12** at a portion of the drainage cannula **10** that does not get inserted into the patient. For example, the valve **13** may be provided in proximity to the outlet fitting **22.** The valve **13** allows a physician or other user to control fluid flow through the first drainage tube **12,** and thereby to control the ratio of fluid drained from the first drainage tube **12** relative to fluid drained from the second drainage tube **14.** Further details of the valve **13** will discussed herein in connection with **FIG. 13****.**

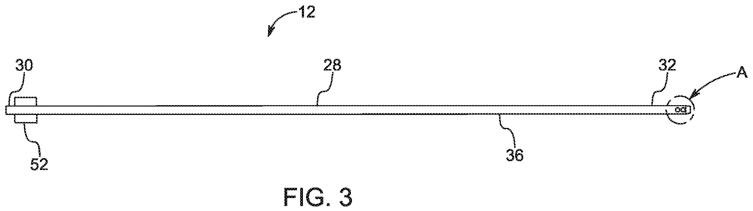

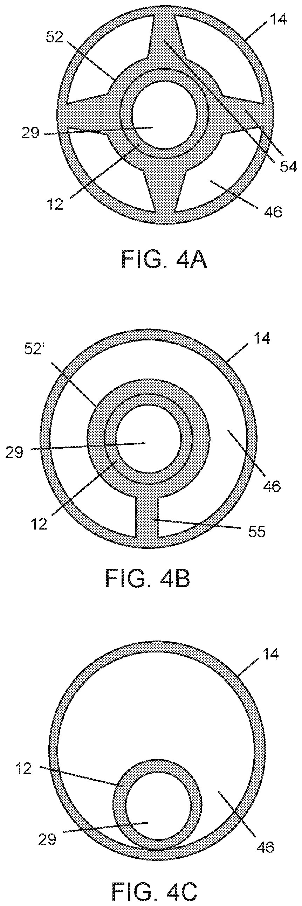

Referring now to **FIGS. 3****,** the first drainage tube **12** of the drainage cannula **10** is illustrated without the second drainage tube **14,** which has been removed for clarity. The first drainage tube **12** has a first elongate body **28** having a working length of, for example, around 40-45 cm. The first elongate body **28** of the first drainage tube **12** is substantially cylindrical and extends from a first proximal end **30** to a first distal end **32.** The first elongate body **28** defines a first lumen **29** (see **FIGS. 4A-5**) extending throughout the entire length of the first drainage tube **12.** In some embodiments, a retainer **52** may extend radially from the first elongate body **28** to position the first drainage tube **12** within the second drainage tube **14.** In some embodiments, the retainer **52** may position the first drainage tube **12** so as to be coaxial with the second drainage tube **14.** In some embodiments, the retainer **52** may be located at or near the first proximal end **30** of the first elongate body **28.** In some embodiments, a plurality of retainers **52** may be spaced along the length of the first elongate body **28.** In still other embodiments, the retainer **52** may extend axially along a portion of the length first elongate body **28** or along the entire length of the first elongate body **28.**

Various embodiments of the retainer **52** will now be described in greater detail with reference to **FIGS. 4A-4C****.** Referring first to **FIG. 4A****,** in some embodiments the retainer **52** may be star-shaped or cross-shaped and may include a plurality of straight or arcuate spokes or legs **54** extending radially between the first drainage tube **12** and the second drainage tube **14.** Fluid in the second drainage tube **14** may flow through the portions of a second lumen **46** defined between the spokes or legs **54.** Referring now to **FIG. 4B****,** in other embodiments the retainer **52'** may include one or more ribs **55** extending radially between the first drainage tube **12** and the second drainage tube **14.** In the embodiments of **FIGS. 4A** and **4B****,** the retainer **52, 52'** may be formed integrally with one or both of the first drainage tube **12** and the second drainage tube **14,** or the retainer **52, 52'** may be a separate component joined to the first drainage tube **12** and/or the second drainage tube **14** during assembly of the drainage cannula **10.** The spokes/legs **54** and/or the ribs **55** of the retainer **52, 52'** may index the first drainage tube **12** at a desired position within the lumen of the second drainage tube **14.** For example, the spokes/legs **54** and/or the ribs **55** may index the first drainage tube **12** so as to be coaxial with the second drainage tube **14.** Referring now to **FIG. 4C****,** in some embodiments the retainer **52** may be omitted and the first drainage tube **12** may be directly connected or integrally formed with the second drainage tube **14.** For example, the wall of the first drainage tube **12** may be in direct contact with the wall of the second drainage tube **14,** such that the first drainage tube **12** is offset from the central longitudinal axis of the second drainage tube **14.**

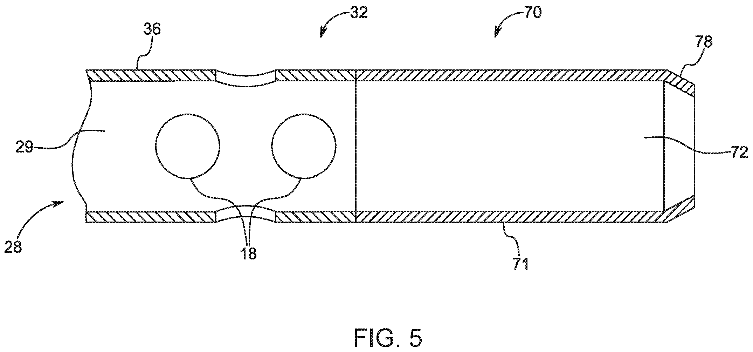

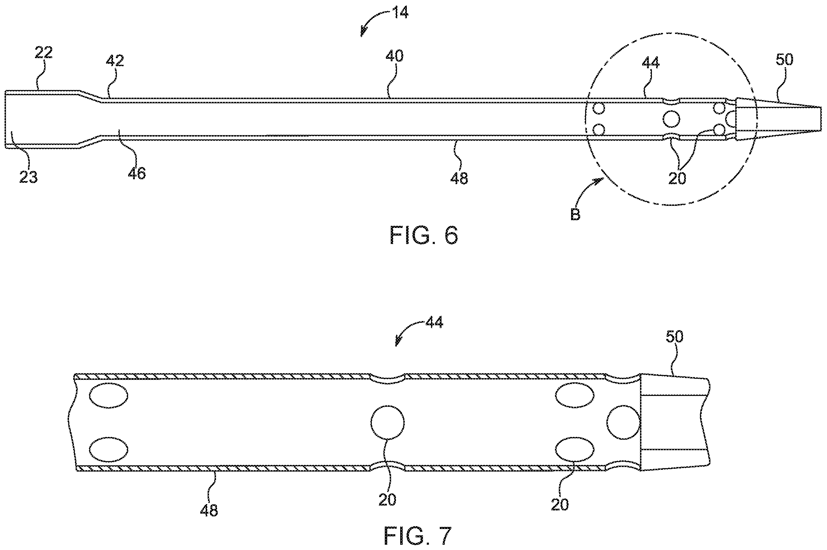

Referring now to **FIG. 5****,** in some embodiments, the first distal end **32** of the first elongate body **28** may be attached to or integrally formed with a pliable distal tip **70** having a hollow structure defined by a tip sidewall **71.** The distal tip **70** defines a tip lumen **72** in fluid communication with the first lumen **29** of the first elongate body **28** to allow fluid flow between the tip lumen **72** and the first lumen **29.** A distal end of the distal tip **70** may include a first tapering section **78** that tapers radially inward in a distal direction for facilitating easier insertion of the first drainage tube **12** into the patient's body. The distal tip **70** may be made from a medical grade material such as polyurethane, or another material suitable for bonding to the material of the first elongate body **28.** In some embodiments, the distal tip **70** may be manufactured from a material having a Shore durometer hardness of less than or equal to 85A to prevent injury and/or discomfort to the patient when the cannula **10** is placed into the patient. In some embodiments, the distal tip **70** may have a Shore durometer hardness of at least 5A less than the Shore durometer hardness of the first elongate body **28.** Other embodiments of the distal tip **70** suitable for use with the drainage cannula **10** of the present disclosure are described in International Application No. PCT/US2019/036987. With reference to **FIGS. 6-7****,** the second drainage tube **14** is illustrated separately from the other components of the drainage cannula **10** for clarity. The second drainage tube **14** has a second elongate body **40** having a working length of, for example, around 30 cm. The second elongate body **40** of the second drainage tube **14** may be substantially cylindrical and extends from a second proximal end **42** to a second distal end **44.** The second elongate body **40** defines the second lumen **46** extending throughout the length of the second drainage tube **14.** The second proximal end **42** may be connected to and/or integrally formed with the outlet fitting **22** (see **FIG. 2**). The second elongate body **40** of the second drainage tube **14** has a hollow structure defined by a second sidewall **48** extending circumferentially about the second elongate body **40.** The second sidewall **48** has a substantially constant thickness throughout the length of the second elongate body **40,** with a second tapering section **50** at the second distal end **44** of the second elongate body **40.** The second tapering section **50** tapers distally to a sidewall thickness of substantially zero to define a smooth transition with the first sidewall **36** of the first drainage tube **12** (see **FIG. 2**) when the drainage cannula **10** is assembled.

With specific reference to **FIG. 7****,** the second distal end **44** of the second drainage tube **14** is shown in detail. The plurality of apertures **20** is provided at the second distal end **44 of** the second drainage tube **14.** The plurality of apertures **20** may extend circumferentially around the second distal end **44.** Each aperture **20** has a diameter of, for example, about 1.5 mm. The plurality of apertures **20** may be arranged in an alternating pattern of axially offset rows around the circumference of the second drainage tube **14.** Each of the plurality of apertures **20** extends through the thickness of the second sidewall **48.** The apertures **20** illustrated in **FIGS. 6** and **7** may extend through the second sidewall **48** in a direction perpendicular to a longitudinal axis of the second elongate body **40.** Alternatively, the plurality of apertures **20** may extend through the thickness of the second sidewall **48** in an angled manner with respect to the longitudinal axis of the second elongate body **40.** For example, the plurality of apertures **20** may be arranged at an acute or obtuse angle with respect to a cross-sectional plane of the second drainage tube **14** extending perpendicular to the longitudinal axis of the second elongate body **40.** In some embodiments, a wire mesh basket (not shown) is provided inside the second lumen **46** of the second drainage tube **14** at a location surrounding the apertures **20.** The wire mesh basket supports and prevents the second sidewall **48** from collapsing due to being weakened by the formation of the plurality of apertures **20.** In some embodiments, one or more sensors (not shown) may be provided at the second distal end **44** of the second drainage tube **14.** The sensor(s) may be adapted for measuring, for example, local blood pressure and/or oxygen concentration.

The total cross-sectional area of the plurality of apertures **20** is desirably approximately equal to or greater than the cross-sectional area of the second lumen **46.** If the cross-sectional area of the plurality of apertures **20** is less than the cross-sectional area of the second lumen **46,** an undesirable pressure drop within the second drainage tube **14** may occur. This pressure drop reduces the flow throughput within the second lumen **46** and impairs the efficiency of the second drainage tube **14.** Desirably, the total cross-sectional area of the plurality of apertures **20** exceeds the cross-sectional area of the second lumen **46** such that if one or more drainage apertures **20** becomes clogged, the total cross-sectional area of the remaining apertures **20** is equal to or greater than the cross-sectional area of the second lumen **46.**

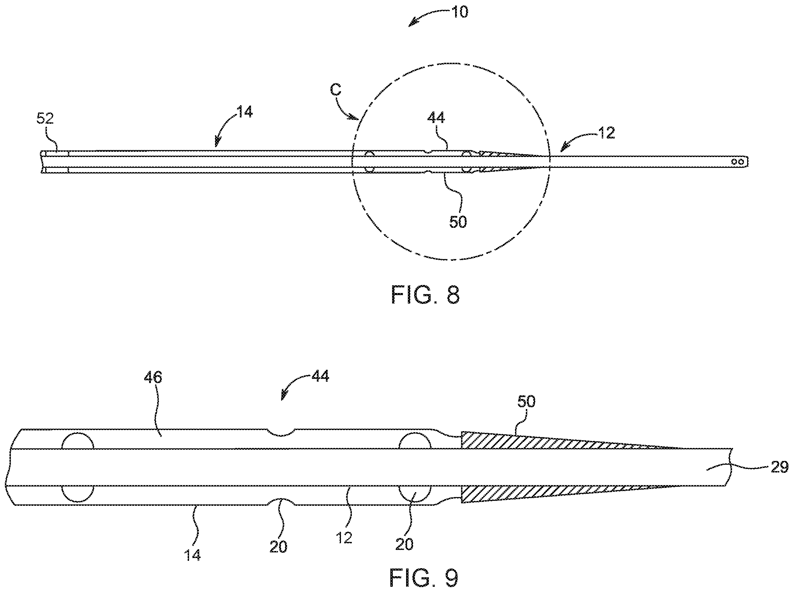

With reference now to **FIGS. 8-9****,** the drainage cannula **10** shown in **FIG. 2** **is** illustrated in cross section. The second distal end **44** of the second drainage tube **14** is fixedly attached to a midportion of the first drainage tube **12** along the length of a second tapering section **50,** as shown in **FIG. 9****.** For example, the second tapered section **50** may be in direct contact with the exterior surface of the first drainage tube **12** such that the circumferential inner surface of the second tapered section **50** of the second drainage tube **14** is secured to the circumferential outer surface of the first drainage tube **12.** The first proximal end **30** of the first drainage tube **12** may be secured to the second drainage tube **14** at or near the second proximal end **42** by the retainer **52.** In the embodiment shown, the first proximal end **30** of the first drainage tube **12** is located distally of the outlet fitting **22.** In other embodiments, the first proximal end **30** of the first drainage tube **12** may extend into the outlet fitting **22.**

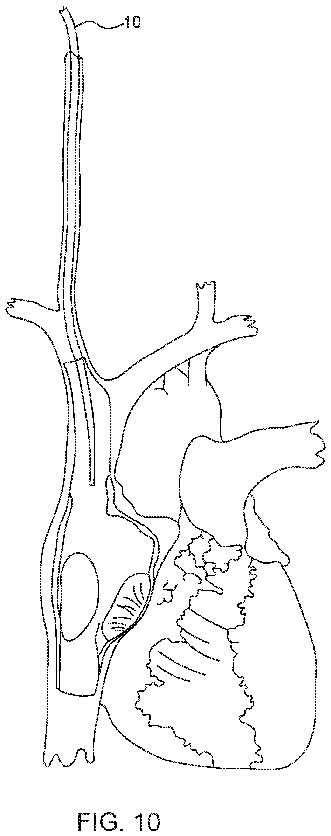

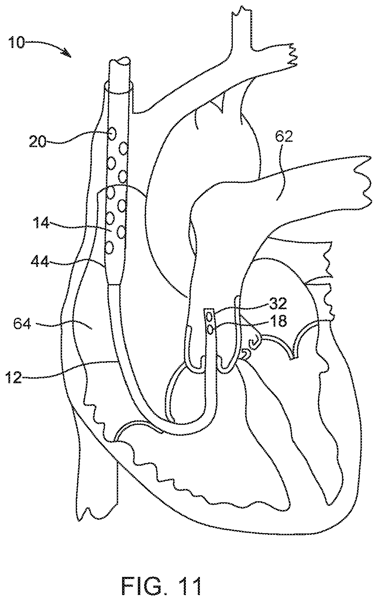

Having described several non-limiting embodiments of the drainage cannula **10** and the connector **22,** an exemplary and non-limiting method for supporting the right heart of a patient using the drainage cannula **10** will now be described with reference to **FIGS. 10-11****.** In use, the drainage cannula **10** is inserted into the pulmonary artery in a percutaneous procedure. Initially, a percutaneous entry needle (not shown) is used to access the patient's internal jugular vein (UV). An introducer, such as a guidewire, is then inserted through the needle until the tip of the introducer is positioned in the upper portion of the inferior vena cava/right atrium (IVC/RA) junction. The needle can then be removed and a pulmonary wedge catheter inserted over the guidewire into the pulmonary artery. The introducer tip is then threaded into the pulmonary artery, and the wedge catheter is removed. The IJV is then serially dilated and the drainage cannula **10** is threaded along the introducer into the IJV, through the right ventricle, and into the pulmonary artery. The first distal end **32** of the first drainage tube **12** is sufficiently flexible about the central axis **16** so as to navigate the IJV, right ventricle, and pulmonary artery. The drainage cannula **10** may include insertion depth markers and radiopaque markers for aiding the user in placing the drainage cannula **10** in the right atrium. Once the position of the drainage cannula **10** is acceptable, the introducer may be removed and the drainage cannula **10** may clamped in place. For example, the drainage cannula **10** may be secured to the patient's neck using a suture. **FIG. 11** shows the drainage cannula **10** positioned in the patient according to some embodiments of the disclosure. In particular, the second distal end **44** is positioned at least within proximity with the right atrium **64,** while the first distal end **32** extends into the pulmonary artery **62.**

Referring now to **FIG. 12****,** the drainage cannula **10** is shown schematically as part of a VA ECMO system **60.** As described above with reference to **FIGS. 10** and **11****,** the first drainage tube **12** is positioned such that the plurality of apertures **18** thereof are located in the pulmonary artery **62,** thereby allowing blood from the pulmonary artery **62** to drain through the plurality of apertures **18** and into the first lumen **29.** The second drainage tube **14** is positioned such that the plurality of apertures **20** thereof are located in the right atrium **64,** thereby allowing blood from the right atrium **64** to drain through the plurality of apertures **20** and into the second lumen **46.** As noted above, in some embodiments, the plurality of apertures **20** may be located in the right ventricle in addition to or as an alternative of being positioned in the right atrium **64,** thereby allowing blood from the right ventricle to drain through the plurality of apertures **20.**

The outlet fitting **22** of the drainage cannula **10** may be connected to an inlet fitting of a blood pump **80.** The pump **80** can be any centrifugal, axial, mixed, or roller pump that can produce adequate flowrates through the system. Several examples of pumps include, without limitation the TANDEMHEART pump manufactured by Cardiac Assist, Inc., the BIOMEDICUS pump manufactured by Medtronic, Inc., the ROTAFLOW pump manufactured by Jostra Medizintechnik AG, the CENTRIMAG pump manufactured by Levitronix, LLC, the SARNS DELPHIN pump manufactured by the Terumo Cardiovascular Group, the REVOLUTION pump manufactured by Cobe Cardiovascular, Inc, and others. The pump **80** can be secured to the patient, for instance with a holster **82** that holds the pump **80** with a strap or in a pocket. The holster **82** can be wrapped around the abdomen or shoulder or leg of the patient. A controller **84** may be provided for controlling the operation of the pump **80.** The controller **84** may be built into the pump **80.** The pump **80** further includes an outlet **86** for delivering blood to an oxygenator **88.** The oxygenator **88** may be secured to the holster **82.** The pump outlet **86 may** be directly connected to the oxygenator **88,** or the pump outlet **86** may be indirectly connected to the oxygenator **88** via a conduit or hose. The oxygenator **88** includes an oxygenation membrane or other element(s) for oxygenating blood flowing through the oxygenator **88.** Oxygenated blood is delivered to an artery in the patient's body through an infusion cannula **90.** While **FIG. 12** illustrates the infusion cannula **90** connected to the patient's femoral artery **92,** the infusion cannula in other embodiments may be connected to the patient's subclavian artery or another artery of the patient's vascular system.

In the VA ECMO system **60,** the drainage cannula **10** allows the right atrial sourcing component, namely the second drainage tube **14,** to drain the majority of venous flow, such as 4 liters per minute (lpm) out of a typical system flow of 5 lpm, leaving the pulmonary artery sourcing component, namely the first drainage tube **12,** to drain the remaining 1 lpm.

Having described several non-limiting aspects of the drainage cannula **10** and the VA ECMO system **60,** an exemplary and non-limiting method for bilateral unloading of a patient's heart using the drainage cannula **10** will now be described with continued reference to **FIGS. 9-12****.**

In use, the drainage cannula **10** is inserted into the patient's vasculature in a percutaneous procedure prior to being connected to the other components of the VA ECMO system **60.** Initially, a percutaneous entry needle (not shown) is used to access the patient's internal jugular vein **94** or the femoral vein. A guidewire, such as a guidewire having maximum diameter 0.038 in. (0.965mm) and a minimum length of 170 cm, is inserted into the vasculature. In some aspects, the positioning of the guidewire is verified using an appropriate imaging technique. In the next step, the patient's active clotting time is checked for approximately 400 seconds.

The drainage cannula **10** may then be guided over the guidewire into the desired position within the patient's vasculature, shown in **FIGS. 11** and **12****.** In some embodiments, an introducer (not shown) may be inserted into the first drainage tube **12** prior to guiding the drainage cannula **10** over the guidewire. In some aspects, the drainage cannula **10 may** be guided into a desired position using indicia, such as a radiopaque marker located in the first distal end **32** of the first drainage tube **12,** that is visualized under fluoroscopy, transthoracic echocardiography, or cineangiography. The position of the drainage cannula **10** may be guided and verified by an imaging system described in WO 2015/139031. After noting and recording the location of the drainage cannula **10,** the introducer (if utilized) can be removed.

To connect the drainage cannula **10** to the blood pump **80,** a wet-to-wet, or other type, of a connection is made between the drainage cannula **10** and the pump **80.** After verifying the correct positioning and insertion depth of the drainage cannula **10,** the drainage cannula **10** can be secured to the patient, such as by suturing with a suture wing. The patient's active clotting time is checked for approximately 180-220 seconds before turning on the blood pump **80** to circulate the patient's blood through the VA ECMO system **60.** During use, fluid drained from the pulmonary artery **62** via the first drainage tube **10** and fluid drained from the right atrium **64** via the second drainage tube **64** flow proximally out of the drainage cannula **10** via the outlet fitting **22** and into the blood pump **80.** The blood pump **80** pumps the blood received from the drainage cannula **10** to the oxygenator **88** to oxygenate the blood, which is then returned to the patient via the infusion line **90.** After use, the pump **80** may be turned off and the pump inlet and outlet may be clamped. Any sutures securing the drainage cannula **10** to the patient may be removed, and the drainage cannula **10** removed from the patient. The puncture site may then be treated and dressed. Additional details of a VA ECMO procedure are described in PCT Application Publication No. WO 2016/054543.

Referring now to **FIG. 13****,** in some embodiments, the valve **13** may be provided on the first drainage tube **12** as discussed herein with reference to **FIG. 2****.** In some embodiments, the valve **13** may be a pinch valve including clamping jaws **15,** a shaft **17,** and a knob **19.** The jaws **15** may be positioned on substantially opposite outer surfaces of the first drainage tube **12.** A physician or other user may rotate the knob **19** to turn the shaft **17,** thereby spreading the jaws **15** apart or drawing the jaws **15** together, as desired. Drawing the jaws **15** together may pinch the first drainage tube **12** to reduce the cross-sectional area of the first lumen **29,** thereby limiting fluid flow through the first drainage tube **12.** In some embodiments, the jaws **15** may be positioned inside the second drainage tube **14,** and the shaft **17** may extend through a sealed hole in the sidewall **48** of the second drainage tube **14** to connect to the knob **19.** While the valve **13 is** shown and described herein as a pinch valve, it should be understood that any suitable type of valve such as a ball valve, a gate valve, or a butterfly valve may be utilized.

## Claims

1. A dual lumen drainage cannula (10) configured for use in a veno-arterial extracorporeal membrane oxygenation (VA ECMO) system (60), the dual lumen drainage cannula (10) comprising:
a first drainage tube (12) having a proximal end, a distal end, and at least one aperture (18) defined in the distal end;
a second drainage tube (14) having a proximal end, a distal end, and at least one aperture (20) defined in the distal end; and
an outlet fitting (22) comprising a single outlet lumen (23) in fluid communication with the first drainage tube (12) and the second drainage tube (14),
wherein the distal end of the second drainage tube (14) is joined to a portion of the first drainage tube (12) between the proximal and distal ends of the first drainage tube (12).

2. The dual lumen drainage cannula (10) according to claim 1, further comprising a retainer for indexing the proximal end of the first drainage tube within the proximal end of the second drainage tube.

3. The dual lumen drainage cannula (10) according to claim 1 or 2, wherein the at least one aperture (18) of the first drainage tube (12) is configured for draining blood from a pulmonary artery of a patient, and
wherein the at least one aperture (20) of the second drainage tube (14) is configured for draining blood from a right atrium of the patient.

4. The dual lumen drainage cannula (10) according to any one of claims 1-3, wherein the first drainage tube (12) extends coaxially relative to the second drainage tube (14).

5. The dual lumen drainage cannula (10) according to any one of claims 1-4, wherein the proximal end of the first drainage tube (12) is positioned distally of the outlet fitting (22).

6. A veno-arterial extracorporeal membrane oxygenation (VA ECMO) system (60) comprising:
the dual lumen drainage cannula (10) of any one of claims 1-5;
a blood pump (80) having an inlet connected to the outlet fitting (22) of the dual lumen drainage cannula (10);
an oxygenator (88) connected to an outlet (86) of the blood pump (80); and
an infusion cannula (90) connected to an outlet of the oxygenator (88) and configured for insertion into the vasculature of a patient.

7. The VA ECMO system according to claim 6, wherein the infusion cannula (90) is configured for insertion into a femoral artery of the patient.

## Patentansprüche

1. Doppellumige Drainagekanüle (10), die zur Verwendung in einem venoarteriellen extrakorporalen Membranoxygenierungs- (VA ECMO)-System (60) ausgestaltet ist, wobei die doppellumige Drainagekanüle (10) umfasst:
ein erstes Drainagerohr (12) mit einem proximalen Ende, einem distalen Ende und mindestens einer Öffnung (18), die in dem distalen Ende definiert ist;
ein zweites Drainagerohr (14) mit einem proximalen Ende, einem distalen Ende und mindestens einer Öffnung (20), die in dem distalen Ende definiert ist;
ein Auslassanschlussstück (22), umfassend ein einzelnes Auslasslumen (23) in Fließkommunikation mit dem ersten Drainagerohr (12) und dem zweiten Drainagerohr (14),
wobei das distale Ende des zweiten Drainagerohrs (14) mit einem Abschnitt des ersten Drainagerohrs (12) zwischen dem proximalen und dem distalen Ende des ersten Drainagerohrs (12) verbunden ist.

2. Doppellumige Drainagekanüle (10) nach Anspruch 1, des Weiteren umfassend einen Halter zum Indexieren des proximalen Endes des ersten Drainagerohrs innerhalb des proximalen Endes des zweiten Drainagerohrs.

3. Doppellumige Drainagekanüle (10) nach Anspruch 1 oder 2, wobei die mindestens eine Öffnung (18) des ersten Drainagerohrs (12) ausgestaltet ist, um Blut aus einer Lungenarterie eines Patienten zu drainieren, und
wobei die mindestens eine Öffnung (20) des zweiten Drainagerohrs (14) ausgestaltet ist, um Blut aus einem rechten Vorhof des Patienten zu drainieren.

4. Doppellumige Drainagekanüle (10) nach einem der Ansprüche 1 bis 3, wobei das erste Drainagerohr (12) sich relativ zu dem zweiten Drainagerohr (14) koaxial erstreckt.

5. Doppellumige Drainagekanüle (10) nach einem der Ansprüche 1 bis 4, wobei das proximale Ende des ersten Drainagerohrs (12) distal zu dem Auslassanschlussstück (22) positioniert ist.

6. Venoarterielles extrakorporales Membranoxygenierungs- (VA ECMO)-System (60), umfassend:
die doppellumige Drainagekanüle (10) nach einem der Ansprüche 1 bis 5;
eine Blutpumpe (80) mit einem Einlass, der mit dem Auslassanschlussstück (22) der doppellumigen Drainagekanüle (10) verbunden ist;
einen Oxygenator (88), der mit einem Auslass (86) der Blutpumpe (80) verbunden ist; und
eine Infusionskanüle (90), die mit einem Auslass des Oxygenators (88) verbunden ist und zur Einführung in das Gefäßsystem eines Patienten ausgestaltet ist.

7. VA ECMO-System nach Anspruch 6, wobei die Infusionskanüle (90) zur Einführung in eine Femoralarterie des Patienten ausgestaltet ist.

## Revendications

1. Canule de drainage à double lumière (10) configurée pour une utilisation dans un système d'oxygénation par membrane extracorporelle veino-artérielle (VA ECMO) (60), la canule de drainage à double lumière (10) comprenant :
un premier tube de drainage (12) ayant une extrémité proximale, une extrémité distale et au moins une ouverture (18) définie dans l'extrémité distale ;
un second tube de drainage (14) ayant une extrémité proximale, une extrémité distale et au moins une ouverture (20) définie dans l'extrémité distale ; et
un raccord de sortie (22) comprenant une seule lumière de sortie (23) en communication fluidique avec le premier tube de drainage (12) et le second tube de drainage (14),
dans laquelle l'extrémité distale du second tube de drainage (14) est assemblée à une partie du premier tube de drainage (12) entre les extrémités proximale et distale du premier tube de drainage (12).

2. Canule de drainage à double lumière (10) selon la revendication 1, comprenant en outre un dispositif de retenue pour indexer l'extrémité proximale du premier tube de drainage à l'intérieur de l'extrémité proximale du second tube de drainage.

3. Canule de drainage à double lumière (10) selon la revendication 1 ou 2, dans laquelle l'au moins une ouverture (18) du premier tube de drainage (12) est configurée pour drainer le sang d'une artère pulmonaire d'un patient, et
dans laquelle l'au moins une ouverture (20) du second tube de drainage (14) est configurée pour drainer le sang d'une oreillette droite du patient.

4. Canule de drainage à double lumière (10) selon l'une quelconque des revendications 1 à 3, dans laquelle le premier tube de drainage (12) s'étend coaxialement par rapport au second tube de drainage (14).

5. Canule de drainage à double lumière (10) selon l'une quelconque des revendications 1 à 4, dans laquelle l'extrémité proximale du premier tube de drainage (12) est positionnée de manière distale par rapport au raccord de sortie (22).

6. Système d'oxygénation par membrane extracorporelle veino-artérielle (VA ECMO) (60) comprenant :
la canule de drainage à double lumière (10) de l'une quelconque des revendications 1 à 5 ;
une pompe à sang (80) ayant une entrée reliée au raccord de sortie (22) de la canule de drainage à double lumière (10) ;
un oxygénateur (88) relié à une sortie (86) de la pompe à sang (80) ; et
une canule de perfusion (90) reliée à une sortie de l'oxygénateur (88) et configurée pour être insérée dans le système vasculaire d'un patient.

7. Système VA ECMO selon la revendication 6, dans lequel la canule de perfusion (90) est configurée pour être insérée dans une artère fémorale du patient.
